# EUROPEAN PATENT APPLICATION

(11) **EP 1 702 576 A1**
(43) Date of publication of application: **20.09.2006**
(21) Application number: 06075309.2
(22) Date of filing: 14.02.2006
(51) Int. Cl.: A61B 17/62, A61B 17/64

(54) **A stabilising system for a hybrid external fixator**

(30) Priority: 16.03.2005 IT MO20050064
(71) Applicant: Rovesti, Gian Luca, 42020 Bibbiano RE (IT)
(72) Inventor: Rovesti, Gian Luca, 42020 Bibbiano RE (IT)
(74) Representative: Gotra, Stefano

(57) **Abstract**

The hybrid external fixator comprises: at least a first element (2), predisposed to be located coaxially of a bone (30); at least a straight second element (3), removably constrainable to the at least a first element (2). Fastening elements (4) are removably associated to both the first and second elements (2, 3), which fastening elements (4) are predisposed for removably fastening to the bone (30); at least a stabiliser bar (5), removably constrainable to the first element (2) and the second element (3) by means for connecting. The means for connecting comprise hooking elements (6), each of which is predisposed to be removably constrained in a seating (7) afforded in the first element (2) or the second element (3), and is conformed in such a way as to lock at least a tract of the stabiliser bar (5) between a hooking element of the hooking elements (6) and the at least a first element (2) or the at least a second element (3).

## Description

External bone fixators are known and usually comprise a first ring-shaped element, normally circular but not necessarily a closed ring, which is predisposed to be located coaxially of a bone and provided with a plurality of through-slots arranged circumferentially. A second element, typically straight or linear, is removably constrained to the circular element. The straight element exhibits a threaded portion which is inserted through the slots of the circular element and is blocked by a nut, so that the straight element is fixed to the annular element in a perpendicular position thereto or with different angulations. Alternatively, the linear element can be constrained to the circular element by a clamp. The straight element is also provided with through-slots, arranged longitudinally, in which fastening elements are removably fixed, such as, for example, screws or Kirschner wires, predisposed to removably fasten the bone to the second element.

In a further embodiment of the invention, not illustrated in the accompanying figures of the drawings, the linear element can be without through-slots. In this case, the bone gripping elements can be fastened to the linear element by clamps. The screws are fixed to the bone by a threaded end, and a constrained to the other end by known-type clamps fastened through the slots, while the wires pass through the bone and are fixed with clamps at both ends.

Two or more fastening elements can be connected parallel to one another to a same straight element, and several straight elements can be associated to the circular element at both sides of the circular element.

The function of external hybrid fixators is to maintain in position two or more portions of a fractured bone. Taking the example of a single-fractured bone, one or more screws can be fixed at an end thereof to each portion of the bone, while at the other end thereof the screws can be fastened to a same straight element or to different straight elements, or to a circular element, according to the conformation of the bone and the position of the fracture. The length and the position of the screws (or wires) enable the two portions of the fractured bone to be maintained in a correct alignment up until the fracture calcifies.

The screws and wires are arranged through the skin and muscle tissues covering the bone and project externally of the broken limb in such a way that the straight element or elements and the circular element are positioned externally of the limb. The circular element is arranged coaxially externally of the limb.

The external fixator, apart from maintaining the fractured portions of bones in correct alignment, must also sustain the loads to which the bone itself is normally subject. Especially in the veterinary field, the fixator has to enable the animal to stay upright and move with relative ease and safety. The external fixator must therefore absorb the load the bone is subject to, protecting the bone from the load and maintaining the bone portions in correct alignment. For this to be possible the fixator must be extremely stable and rigid.

The critical points for stability and rigidity of the external hybrid fixator are constituted by the connection zones between the circular element and the straight elements. If the connection between the elements is realised only by means of fastening lock nuts on the threaded ends of the straight elements, all of the forces absorbed by the fixator tend to unload on the connection zones. This leads to a very high risk of breakages of the fixator due to excessive mechanical fatigue. To avoid this risk of fixator breakage a support is necessary, which reduces the loads exerted on the connection zones between the straight elements and the circular element.

To this end fixators of known type are provided with stabiliser bars which are constrained between the circular element and each of the straight elements associated thereto. The bars are fixed to the circular element and to the respective straight element by special clamps. The clamps exhibit various anchoring types to the straight and circular elements, and an orientable head having means for blocking the stabiliser bars. The clamps are relatively unwieldy, and considerably complicate the structure of the hybrid external fixator, lengthening fixator mounting operations. It is often decidedly difficult to find a suitable position for all of the parts making up the fixator: the anchoring clams for the bolts and the wires frequently interfere with the stabiliser bars, while the fastening clamps for the stabiliser bars interfere with the bolts and the wires, and create a need for lengthy and complicated manoeuvring in order to find an acceptable position for each element. Often the mass caused by the presence of all the described elements is incompatible with the radiographic screens, which are needed to monitor the quality of the mounting and the progress of the bone healing process.

The main aim of the present invention is to provide a hybrid external bone fixator which is stable and resistant to mechanical stress following installation thereof.

A further aim of the present invention is to provide a hybrid external fixator which is compatible with the instruments commonly used for external bone fixation, especially in the field of veterinary practice, in order to avoid the need for using specific instruments in addition to commonly-used instruments.

A further aim of the invention is to provide a hybrid external fixator which is easy and rapid to install, so that it has a minimum impact on surgical operation times, and which is light and easily adaptable to various installation configurations.

A further aim of the invention is to provide a hybrid external fixator which only very slightly influences availability of radiographic access, necessary for post-operative monitoring of the quality of the mount and progress of the healing process.

Further characteristics and advantages of the present invention will better emerge from the detailed description that follows, made with reference to the accompanying figures of the drawings, provided by way of non-limiting example, in which:
Figure 1 is an overall perspective view of a fixator of the present invention, in a use configuration;
Figures 2 and 3 show a plan view of two elements of the fixator of figure 1;
Figure 4 is a sectioned detail of the element of figure 2.

With reference to the figures of the drawings, the hybrid external fixator of the present invention comprises at least a first annular element 2, normally termed circular, which is predisposed to be located coaxially of a bone 30. The first annular element 2, which has a closed-ring conformation, or is an arc of a circumference, but is not necessarily geometrically circular in the strict sense, is delimited by two base surfaces 21 which are parallel to one another and connected by two concentric lateral surfaces 23, 24. The first element 2 is provided with through-slots 2a, arranged circumferentially, which extend between the base surfaces through the whole width of the first element 2. The through-slots 2a are separated from one another by full portions of the first element 2.

At least a second element 3, preferably straight, is removably constrainable to the first element 2. The straight second element 3 is provided with through slots 3a, arranged transversally to the longitudinal development of the second element 3, which extend through the width of the second element 3 between two parallel and opposite lateral surfaces 31. The through-slots 3a are at least partially delimited and separated from one another by full portions of the second element 3. In a further embodiment of the invention, not illustrated in the accompanying figures of the drawings, the linear element can be without the through-slots. The second element 3 is further provided with a threaded limb 32 predisposed to be inserted through a through-slot 2a of the first element 2. Using a nut screwed on the threaded limb 32 the second element 3 is fastened to the first element 2. Alternatively the fastening of the straight element to the annular element can be done using a special clamp.

Fastening elements 4, predisposed to fasten the second element 3 to the bone 30, are removably associable with the second element 3. The fastening elements 4 can be constituted by straight screws of bolts which, in a known way, are constrained at one end thereof to the bone 30 and at the other end thereof to the second element 3 by clamps 12. The clamps 12 are usually provided with a threaded limb, which is inserted and fastened in a through-slot 3a by means of a nut, and a head exhibiting known means for gripping and blocking the screws or bolts.

At least a stabiliser bar 5 is removably constrainable to the first and second element 2, 3 by means for connecting. The means for connecting comprise hooking elements 6, each of which is predisposed to be removably constrained in a seating 7 afforded in the first element 2 or the second element 3. Each hooking element is conformed so as to lock at least a tract of the stabiliser bar 5 between itself and the first element 2 or the second element 3.

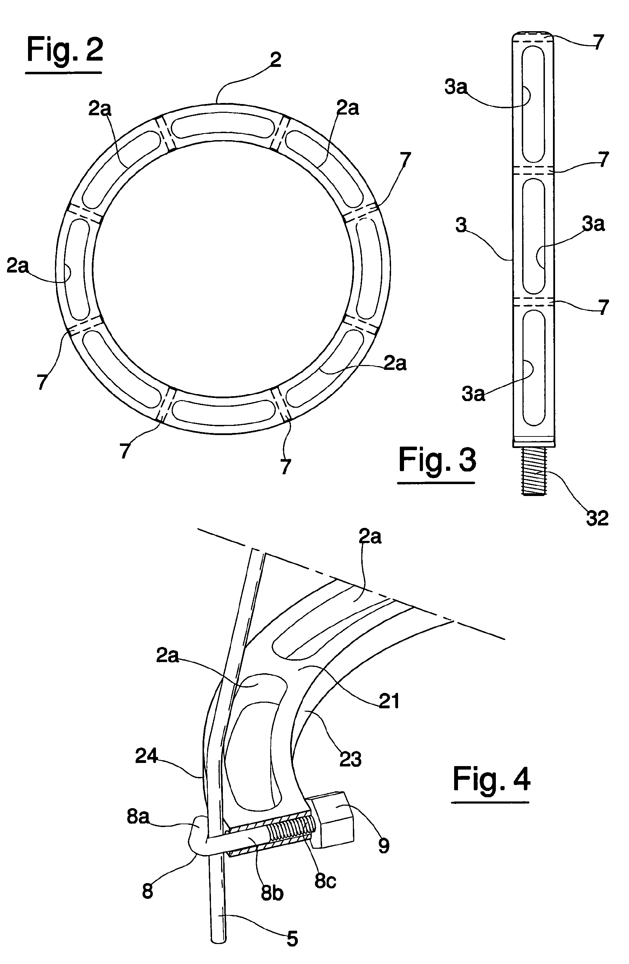

Each of the hooking elements (figure 4) is preferably defined by a hook 8 having a straight portion 8b and a bent portion 8a. Preferably each hook 8 is further provided with a threaded limb 8c, aligned to the straight portion 8b and predisposed to screw into a nut 9.

Each of the seatings 7 is preferably defined by a through-hole arranged radially through the second element 3. In particular, the seatings 7 are afforded through the full portions which separate the through-slots 2a, 3a of the first element 2 and the second element 3. In the first element 2, the seatings 7, or holes, extend between the lateral surfaces of the first element 2 along a radial direction which passes through the full portions that separate the through-slots 2a. In the second element 3, the seatings 7, or holes, extend parallel to the lateral surfaces 31 of the second element 3 between which the through slots 3a are located. In a further embodiment of the invention, not illustrated, the straight element can be without the through slots.

Each hook 8 can be slidably inserted in a respective seating 7, so that the bent end 8a projects from an end of the seating 7 itself, while the threaded limb 8c projects from the opposite end of the seating 7. Each hook 8 is slidable between at least a non-operative position and an operative position. In the non-operative position the bent end 8a is distanced from the lateral surface affording the seating 7 of the first element 2 or the second element 3 and a tract of the stabiliser bar 5 can be easily positioned between the lateral surface of the first element 2 or the second element 3 and the bent end 8a. In the operative position the bent end 8a locks the above-mentioned tract of the stabiliser bar 5 against the lateral surface of the first element 2 or the second element 3. The blocking of each hook 8 in the operative position is obtained by a respective nut 9 which is screwed on the threaded limb 8c of the hook 8 and locked on the opposite lateral surface with respect to the surface from which the bent end 4a projects. Other means for blocking can however be included for performing the function of blocking and gripping the hooks 8 in the operative position.

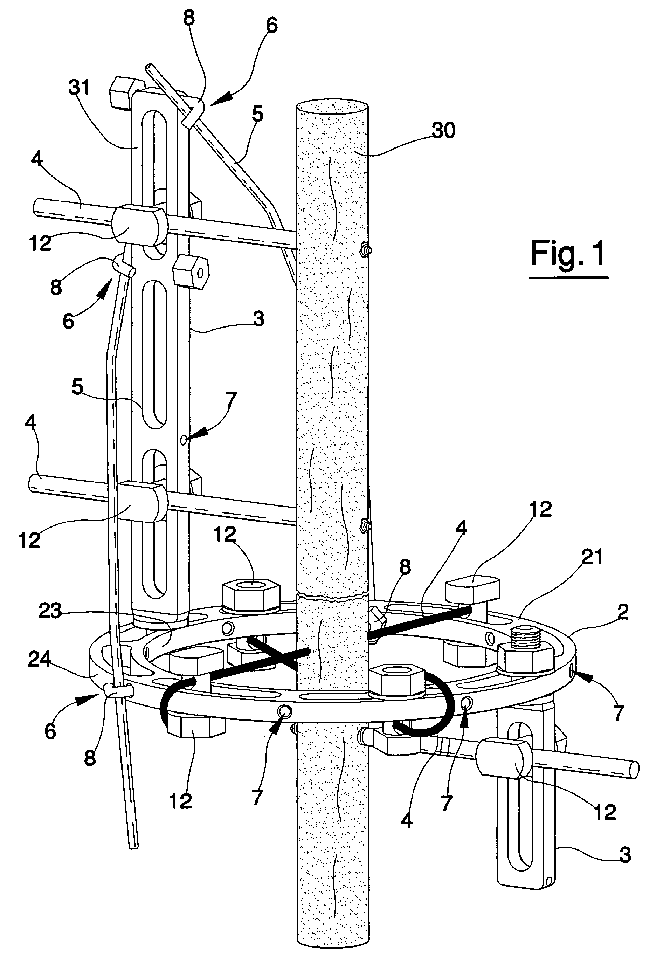

An example of an operative configuration of the fixator according to the invention is illustrated in figure 1. A second element 3 is associated to the first element 2 and stabilised by two stabiliser bars 5 arranged on opposite sides with respect to the second element 3. Each bar 5 functions as a bolt and is constrained by two hooks 8 located in respective seatings 7, one positioned in the first element 2 and one in the second element 3. Selecting different positions for the hooks 8, however, it would have been possible to position the bars 5 differently. Different positions can be realised also by selecting stabiliser bars of different lengths. Several straight second elements 3 can be associated to the first element 2 on the same side or on opposite sides of the first element 2. Each straight second element 3 can be stabilised by means of respective stabiliser bars 5 which can be located in a predetermined position by choosing a suitable position for the hooks 8.

Stabilising the fixator mounting is much more rapid than installing a known-type fixator. Once the fixator has been mounted and fixed to the fractured bone, stabilising the second element 3 (and all the other linear elements which may be present) occurs by identifying two seatings 7, one on the second element 3 and one on the first element 2, which can be connected by a bar 5. The bar 5 is anchored to the first element 2 and the second element 3 by two hooks 8 which block the bar 5 in the desired position and which are fastened using nuts 9 tightened on the threaded limbs 8c.

Advantageously, to facilitate installation of the fixator, the stabiliser bar 5 (or bars) is at least partially deformable. In a case where a clamp of the fastening elements 4 creates an interference to the positioning of a stabiliser bar 5, at moment of installation the bar 5 can be directly slightly bent and adapted to pass laterally of the clamp. Since the bars 5 work by traction, a slight flexion thereof will not cause problems of instability. Preferably the stabiliser bars 5 are made of malleable steel, while the first element 2 and the second element 3 are made of aluminium alloy, but any material exhibiting sufficiently malleable and resistant characteristics can be used for realising the stabiliser bars 5.

The transversal section of the stabiliser bars 5 is preferably circular and exhibits suitable dimensions for enabling a locking of the bars 5 by means of the bent portion 8a of the hooks 8.

The hybrid external fixator of the present invention attains all of the set aims. It is light, stable and resistant to mechanical stresses subsequent to installation thereof. It is also easy and rapid to install, and thus scarcely has an effect on surgical operation times. It is also easily adaptable to various use configurations. As it is of limited size, the fixator has a very limited influence on the availability of radiographic spaces which are necessary for post-operative evaluation of the quality of mounting and of the progress of the bone healing.

## Claims

1. A hybrid external fixator, comprising:
at least a first element (2), having a closed-ring annular or arc of circumference conformation, predisposed to be located coaxially of a bone (30);
at least a second element (3), removably constrainable to the at least a first element (2), to which fastening elements (4) are removably associated, which fastening elements (4) are predisposed for removably fastening the at least a second element (3) to the bone (30);
at least a stabiliser bar (5), removably constrainable to the at least a first element (2) and the at least a second element (3) by means for connecting;
**characterised in that** the means for connecting comprise hooking elements (6), each of which is predisposed to be removably constrained in a seating (7) afforded in the at least a first element (2) or the at least a second element (3), and is conformed in such a way as to lock at least a tract of the at least a stabiliser bar (5) between a hooking element of the hooking elements (6) and the at least a first element (2) or the at least a second element (3).

2. The hybrid external fixator of claim 1, **characterised in that** each of the hooking elements (6) is defined by a hook (8) having a straight portion (8b) and a bent portion (8a).

3. The hybrid external fixator of claim 1, **characterised in that** each of the seatings (7) is defined by a through-hole arranged radially in the at least a first element (2) or transversally in the at least a second element (3).

4. The hybrid external fixator of claim 2 and 3, **characterised in that** each hook (8) is slidably insertable in a respective seating (7) in such a way that the bent end (8a) thereof projects externally of the seating (7) itself, each hook (8) being slidable between at least a non-operative position, in which the bent end (8a) is distanced from a lateral surface on which the seating (7) is located in the at least a first element (2) or the at least a second element (3), and a tract of the stabiliser bar (5) can be positioned between the lateral surface and the bent end (8a), and an operative position, in which the bent end (8a) locks the tract of the stabiliser bar (5) against the lateral surface.

5. The hybrid external fixator of claim 4, **characterised in that** it comprises means for blocking predisposed to activate the hooks (8) in a sliding motion and lock the hooks (8) in the operative position.

6. The hybrid external fixator of claim 5, **characterised in that** the means for blocking comprise a threaded end (8c) of the hooks (8) predisposed to screw into a nut (9).

7. The hybrid external fixator of any one of the preceding claims, **characterised in that** the at least a first element (2) is provided with through-slots (2a) arranged circumferentially and separated by full portions of the at least a first element (2), in which the seatings (7) are afforded.

8. The hybrid external fixator of any one of the preceding claims, **characterised in that** the at least a second element (3) is provided with through-slots (3a) arranged transversally of a longitudinal development of the at least a second element (3), the through-slots (3) being delimited and separated from one another by full portions of the at least a second element (3), in which full portions the seatings (7) are afforded.

9. The hybrid external fixator of any one of the preceding claims, **characterised in that** the stabiliser bar (5) is at least partially deformable.

10. The hybrid external fixator of any one of claims from 2 to 9, **characterised in that** the stabiliser bar (5) exhibits a transversal section having a size which is suitable for enabling a locking of the stabiliser bar (5) by means of the bent portion (8a) of the hooks (8).
